# FASCICULE DE BREVET EUROPEEN

(11) **EP 3 052 201 B1**
(45) Date de publication et mention de la délivrance du brevet: **06.12.2017**
(21) Numéro de dépôt: 14783573.0
(22) Date de dépôt: 01.10.2014
(51) Int. Cl.: A61Q 19/00, A61K 8/92, A61K 8/97

(54) **UTILISATION D'UNE COMPOSITION HUILEUSE CONTENANT UN EXTRAIT D'HEMEROCALLE POUR AMELIORER L'ECLAT DU TEINT**
VERWENDUNG VON EINER ÖLIGEN ZUBEREIZUNG ENTHALTEND EINEN HEMEROCALLIS-EXTRAKT ZUR VERBESSERUNG DER AUSSTRAHLUNG DES TEINTS
USE OF AN OILY COMPOSITION COMPRISING AN EXTRACT OF HEMEROCALLIS FOR FOR ENHANCING THE RADIANCE OF THE SKIN'S COMPLEXION

(30) Priorité: 02.10.2013 FR 1359548
(43) Date de publication de la demande: 10.08.2016
(73) Titulaire: Société De Recherche Cosmétique S.à.r.L., 2314 Luxembourg (LU)
(72) Inventeur: LECONTE, Nadine, F-92600 Asnieres Sur Seine (FR); ROSSIGNOL-CASTERA, Anne, F-34160 Restinclieres (FR)
(74) Mandataire: Goulard, Sophie
(86) Numéro de dépôt international: PCT/EP2014/071007
(87) Numéro de publication internationale: WO 2015/049266

(56) Documents cités:
- FR-A1- 2 943 684
- JP-A- 2006 143 670
- JP-A- 2012 012 318
- DATABASE GNPD [Online] MINTEL; novembre 2009 (2009-11), KAO: "Cream est Eternal Flow", XP002722293, Database accession no. 1211158
- DATABASE GNPD [Online] MINTEL; juillet 2013 (2013-07), Mantong, South Korea: "Transformation Cleansing Milk Oil", XP002722302, Database accession no. 2122962
- NAMSA N D ET AL: "An ethnobotanical study of traditional anti-inflammatory plants used by the Lohit community of Arunachal Pradesh, India", JOURNAL OF ETHNOPHARMACOLOGY, ELSEVIER IRELAND LTD, IE, vol. 125, no. 2, 7 septembre 2009 (2009-09-07), pages 234-245, XP026498478, ISSN: 0378-8741, DOI: 10.1016/J.JEP.2009.07.004 [extrait le 2009-07-14]

## Description

La présente invention se rapporte au domaine technique général des produits cosmétiques à usage topique, utilisables notamment sur la peau.

Plus précisément, la présente invention est relative à l'utilisation d'une composition huileuse à base d'un extrait lipophile d'hémérocalle à titre d'ingrédient actif pour la préparation d'une composition cosmétique à usage topique destinée à améliorer l'éclat du teint et/ou uniformiser le teint, à un procédé de traitement cosmétique non thérapeutique de la peau mettant en oeuvre une telle composition cosmétique, ainsi qu'à l'utilisation non thérapeutique d'une composition cosmétique à application topique comprenant un extrait lipophile d'hémérocalle pour améliorer l'éclat du teint et/ou uniformiser le teint.

La peau est un véritable organe comprenant plusieurs couches intégrées, allant de la couche superficielle, l'épiderme, jusqu'aux couches plus profondes, le derme et l'hypoderme, et chacune de ces couches possède des propriétés spécifiques permettant à l'ensemble de réagir et de s'adapter aux conditions de son environnement.

L'épiderme est principalement composé de kératinocytes (90% des cellules épidermiques) qui synthétisent la kératine, de mélanocytes (2 à 3% des cellules épidermiques) responsables de la pigmentation de la peau, et des cellules de Langerhans qui jouent un rôle immunologique. L'épiderme, dont l'épaisseur est variable selon les différentes parties du corps, constitue la couche externe de la peau et par conséquent il joue un rôle fondamental pour assurer la protection et le maintien d'une bonne trophicité. C'est pourquoi de nombreuses compositions ont été mises au point afin de le protéger, notamment des agressions de l'environnement.

Le derme est la couche la plus épaisse, riche en nerfs, en vaisseaux sanguins et en glandes sudoripares, et se compose principalement de collagène, d'élastine et de protéoglycanes. Ces trois types de molécules sont synthétisés par les fibroblastes dermiques. Les fibres de collagène, qui représentent 70% du poids sec du derme, assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

La notion d'éclat du teint est très complexe, et d'origine multifactorielle. L'éclat du teint peut être défini comme résultant du mélange pondéré des caractéristiques de la texture de la surface cutanée, de sa structure de sa brillance, de sa microcirculation et de sa couleur et en particulier l'uniformité de cette couleur.

L'uniformité du teint et son éclat sont une problématique qui n'est plus seulement réservée aux pays asiatiques mais également à toutes les populations occidentales.

La lipofuscine est un pigment cellulaire jaune-brun formé par la liaison de lipides et de protéines, issue de la peroxydation des acides gras membranaires dans des cellules vieillissantes (produits de glycation des protéines ou selon l'expression anglophone *«AGE-Pigment»* pour *«Advanced Glycation End-product »*).

Ainsi, avec l'âge, on observe la formation de lipofuscine, notamment au niveau de la peau, ce qui a pour effet de ternir le teint, lui conférant une composante jaune au dépend de la composante rosée témoin de la jeunesse et d'une bonne santé. Une accumulation de lipofuscine dans les cellules cutanées peut aussi être responsable de l'apparition de tâches brunes également appelées tâches de vieillesse.

Il est possible de palier l'accumulation de lipofuscine dans les cellules cutanées par l'application sur la peau de compositions cosmétiques contenant un ou plusieurs actifs ayant la capacité de dégrader la lipofuscine et/ou d'empêcher ou ralentir sa formation, par exemple en freinant la glycation des protéines.

Les produits d'origine naturelle ou contenant des composés naturels sont par ailleurs de plus en plus appréciés par les consommateurs et consommatrices de produits cosmétiques. C'est pourquoi on trouve sur le marché des produits cosmétiques de plus en plus de produits contenant des substances naturelles ou d'origine naturelle.

De telles compositions contiennent classiquement au moins une substance anti-oxydante, en particulier des molécules complexes d'origine végétale telles que par exemple les polyphénols, les flavonoïdes, les anthocyanosides, les caroténoïdes, etc. En particulier, les feuilles de l'olivier par exemple, qui sont riches en oleuropéine ont une action sur la lipofuscine. D'autres extraits végétaux tels que ceux de issus de l'arbre à soie (*Albizia julibrissin*), du café grâce à leur composition phénolique, ou encore la fraction éthanolique de mélisse ont une action sur la glycation des protéines. Ces molécules ne sont toutefois pas toujours assez stables dans le temps et perdent ainsi progressivement leur efficacité.

Il est donc difficile, à l'heure actuelle, d'obtenir des compositions cosmétiques contenant un extrait végétal d'origine naturelle et dont les propriétés restent stables dans le temps, alors même que le besoin semble important.

Hémérocalle est le nom commun donné à la fleur du genre *Hemerocallis* qui appartient à la famille des liliacées. Elle est aussi appelée Lys d'un jour. En effet, elle doit son nom au grec ήµέρα (Hemera) «jour» et καλóς (kalos) «beauté». On l'appelle lys d'un jour, car sa fleur est éphémère et ressemble au lys, mais le feuillage est totalement différent. La fleur de l'hémérocalle ne vit qu'une seule journée, mais de nouvelles fleurs se succèdent pendant plusieurs semaines. Les fleurs se fanent à la tombée du jour et laissent la place à d'autres dès le lendemain matin.

Les Hémérocalles sont connues et cultivées depuis des siècles. Originaires d'Asie, les premières espèces étaient déjà cultivées, décrites et peintes à l'époque de Confucius (551 - 479 Av JC).

*Hemerocallis fulva* est connue, notamment en médecine traditionnelle chinoise, pour ses effets calmants, cicatrisants et bactéricides. Elle est également utilisée dans le traitement de la dépression, de l'inflammation, de l'insomnie et des furoncles.

En particulier, la demande de brevet US2011/0076349 décrit une composition à administrer par voie orale contenant, à titre de principe actif, un extrait aqueux des parties aériennes *d'Hemerocallis fulva* et ayant des effets antidépresseurs ou défatiguants basés sur l'amélioration du sommeil.

Les études effectuées par la Demanderesse ont montré qu'un extrait huileux d'Hémérocalle, plus précisément *d'Hemerocallis fulva,* induit une diminution de la quantité de lipofuscine à la fois dans le *stratum granulosum* et dans le reste de l'épiderme. Sans vouloir être liés par une quelconque théorie, les inventeurs de la présente demande pensent que cet effet est dû à la présence dans cet extrait d'une quantité très importante de molécules anti-oxydantes en particulier d'acides chlorogéniques, permettant de diminuer la quantité de protéines oxydées.

La présente invention a donc pour premier objet l'utilisation, à titre d'ingrédient actif, d'une composition huileuse à base d'un extrait lipophile *d'Hemerocallis fulva* et d'un véhicule huileux comprenant au moins une huile végétale (huile vectrice), pour la préparation d'une composition cosmétique à usage topique destinée à améliorer l'éclat du teint et/ou à uniformiser le teint, notamment par la suppression de la composante jaune du teint, ladite composition huileuse représentant de 0,01 à 2 % en masse par rapport à la masse totale de ladite composition cosmétique.

L'invention a également pour objet un procédé cosmétique non thérapeutique pour améliorer l'éclat du teint et/ou uniformiser le teint, consistant à appliquer sur les zones de la peau concernées au moins une composition cosmétique topique contenant de 0,01 à 2 % en masse d'une composition huileuse à base d'un extrait lipophile *d'Hemerocallis fulva* et d'un véhicule huileux comprenant au moins une huile végétale.

Enfin, l'invention a également pour objet l'utilisation non thérapeutique d'une composition cosmétique à application topique comprenant de 0,01 à 2 % en masse d'une composition huileuse à base d'un extrait lipophile *d'Hemerocallis fulva* et d'un véhicule huileux comprenant au moins une huile végétale, pour améliorer l'éclat du teint et/ou uniformiser le teint.

Selon une forme de réalisation préférée de l'invention, la teneur en composition huileuse dans la composition cosmétique varie de 0,1 à 2% en masse et encore plus préférentiellement de 0,5 à 1,5 % en masse par rapport à la masse totale de la composition cosmétique.

Selon une forme de réalisation encore plus préférée, la composition huileuse représente 1 % en masse par rapport à la masse totale de la composition cosmétique.

Les compositions cosmétiques à application topique comprenant une telle composition huileuse à titre d'ingrédient actif présentent d'excellentes propriétés anti-oxydantes utilisables en cosmétique pour améliorer l'éclat du teint et/ou uniformiser le teint, notamment en diminuant ou en supprimant la composante jaune du teint qui est apportée par la présence d'une accumulation de lipofuscine dans les cellules cutanées. Les propriétés anti-oxydantes des compositions cosmétiques incorporant la composition huileuse conforme à l'invention sont de plus particulièrement stables dans le temps.

Les essais effectués par la Demanderesse ont montré qu'un extrait lipophile d'Hémérocalle, plus précisément *d'Hemerocallis fulva,* induit une diminution de la quantité de lipofuscine à la fois dans le *stratum granulosum* et dans le reste de l'épiderme sur des épidermes reconstruits et que cette activité est particulièrement prononcée lorsque la quantité de composition huileuse est inférieure à 4 % en masse, cet effet étant tout particulièrement marqué lorsque la quantité de composition huileuse est voisine de 1 % en masse. En effet, la composition huileuse conforme à l'invention est composée d'une combinaison d'antioxydants qui agissent de façon synergique contre les phénomènes oxydatifs auxquels les cellules cutanées sont exposées, en particulier contre la glycation des protéines conduisant à la formation des produits finaux de glycation parmi lesquels figure le pigment lipofuscine. Elle apporte à la peau à la fois des antioxydants apolaires tels que la vitamine E et des caroténoïdes (lutéine, zéaxanthine), mais aussi des antioxydants plus polaires tels que les polyphénols dont les acides chlorogéniques en quantité importante. Cette combinaison d'agents antioxydants, véhiculée par la ou les huiles végétales du véhicule huileux, est particulièrement efficace pour améliorer l'éclat du teint et/ou uniformiser le teint.

De plus, aux doses utilisées, les essais effectués ont montré que la composition huileuse utilisée dans la présente invention ne présente aucune cytotoxicité.

Les études effectuées par la demanderesse ont montré que l'on peut utiliser toutes les parties aériennes de la plante, aussi bien les tiges, les feuilles et les fleurs. L'espèce *Hemerocallis fulva* est facilement disponible et la conservation des plantes ne soulève généralement pas de difficulté technique.

La composition huileuse conforme à l'invention peut être préparée selon le procédé d'extraction décrit dans la demande internationale WO 2010/112760 consistant, dans une première étape, à mélanger et imprégner la plante, préalablement séchée et broyée, dans au moins une huile végétale naturelle à une température supérieure à son point de fusion, puis à chauffer le mélange à haute température pendant une durée très courte, et à former une microdispersion à une température supérieure à la température de fusion de l'huile végétale. Toutes ces étapes se font de préférence à l'abri de l'air, plus particulièrement à l'abri de l'oxygène pour éviter toute réaction d'oxydation de l'extrait. On peut par exemple travailler sous atmosphère d'azote, dans un réacteur clos avec extraction continue de l'oxygène par un flux d'azote, ou encore sous vide.

Les huiles végétales naturelles utilisables dans ce procédé sont de préférence choisies parmi l'huile d'avocat, l'huile de rosier muscat, l'huile de noisette, l'huile de colza, l'huile de macadamia, l'huile d'argan, l'huile de tournesol, l'huile de cameline, l'huile de bourrache, l'huile d'amande douce, l'huile de carthame, l'huile de calophyllum et leurs mélanges.

Selon, une forme de réalisation préférée de l'invention, l'huile végétale est choisie parmi l'huile d'avocat, l'huile de rosier muscat et leurs mélanges.

Selon une forme de réalisation tout particulièrement préférée de l'invention, le véhicule huileux est un mélange d'huile d'avocat et d'huile de rosier muscat.

Les huiles végétales utilisées selon l'invention, et en particulier les huiles d'avocat et de rosier muscat, sont des produits disponibles dans le commerce.

L'avocat est le fruit de l'avocatier (*Persea americana*), un arbre originaire d'Amérique du Nord (Mexique). Riche en acide oléique, en caroténoïdes et en éléments nutritifs, l'huile d'avocat est extraite de la pulpe du fruit (chair) car paradoxalement, le noyau ne contient que très peu de corps gras.

L'huile de rosier muscat est extraite des graines du cynorhodon (un « faux » fruit) du rosier muscat qui est un églantier sauvage. Elle est l'une des huiles végétales les plus riches en acides gras poly-insaturés essentiels (acides linoléique et linolénique) et contient des insaponifiables. Elle a des propriétés régénérantes.

L'utilisation de ces huiles végétales comme solvant de l'extrait de d'hémérocalle présente l'avantage d'être compatible avec les normes des produits certifiés Ecocert s'appliquant aux produits cosmétiques à la fois écologiques et biologiques.

La première étape du procédé ci-dessus, consistant à imprégner la plante, de préférence broyée, se déroule à température ambiante, ou à chaud, à une température supérieure à celle de fusion des huiles utilisées. On travaille de préférence à température ambiante, de l'ordre de 25°C, les huiles végétales, et en particulier les huiles d'avocat et de rosier muscat, étant liquides à cette température.

Selon une forme de réalisation préférée, le broyage de la plante est effectué à une température d'environ -80°C, jusqu'à obtention d'une poudre présentant une granulométrie moyenne inférieure à 100 µm environ.

La deuxième étape comporte un chauffage à une température élevée, par exemple de l'ordre de 140 à 170°C, pendant une courte durée, généralement inférieure à 5 minutes. Le temps de montée en température doit aussi être très court. La technique du chauffage par micro-ondes est bien adaptée à ces conditions. La technique des micro-ondes facilite aussi la troisième étape de microdispersion des particules dans la ou les huiles végétales utilisées, avec rupture des membranes cellulaires. Cette troisième étape peut aussi comprendre un traitement par cavitation ultrasonore, de préférence dans un réacteur fermé équipé d'un générateur d'ultrasons à basse fréquence. Cette troisième étape peut éventuellement être réalisée simultanément avec les deux autres, l'ensemble des étapes, ou chacune d'elles, pouvant être réalisé plusieurs fois si nécessaire.

A la fin de l'extraction, l'extrait lipophile est de préférence filtré sur un filtre ayant une porosité inférieure à environ 5 µm, de façon à obtenir un extrait lipophile limpide.

Ce procédé est particulièrement efficace sur une matière première telle que l'hémérocalle contenant entre 85 et 90 % d'eau avant broyage et séchage. Après séchage, avant l'étape d'imprégnation dans la ou les huiles végétales, la teneur en eau est ramenée en dessous de 12 %.

Lorsque l'extraction est réalisée en utilisant un mélange d'huile d'avocat et de rosier muscat, on obtient un extrait lipophile de couleur vert foncé ayant une densité relative à 20°C variant de 0,900 à 0,990 g/cm³ environ (NF ISO 6883), soluble dans les huiles et phases grasses, et ayant les caractéristiques suivantes :
- Teneur en eau et en volatils (NF EN ISO 662) : max. 0,20 % ± 0,04 % ;
- Acidité oléique (NF EN ISO 660) : max. 3,0% ± 0,6% ;
- Indice de peroxyde (NF EN ISO 3960) : max 10,0 ± 4,0 méqO₂/kg ;
- Teneur en phénols (dosages par HPLC) :
   * Catéchines : 150 mg/kg
   * Quercétine : 250 mg/kg
   * Rutine : 150 mg/kg
   * Acide chlorogénique : 416 mg/kg.
- Teneur totale en caroténoïdes (lutéine et zéaxanthine) : 5,1 mg/kg de composition huileuse (mesuré par HPLC) ;
- Teneur en β-carotène : 8,6 mg/kg de composition huileuse (mesuré par HPLC) ;
- Composition en acides gras de la composition huileuse :
   * acide palmitique 6-26 %
   * Acide palmitoléïque 1,5-10 %
   * Acide stéarique max. 2 %
   * Acide oléique 30-65 %
   * Acide linoléique 13-27 %
   * Acide α-linolénique 5-14%
   * Acide arachidique max. 1 %
   * Acide érucique max. 1 %

De plus, la technique utilisée permet d'obtenir une composition huileuse bien plus concentrée en principes actifs qu'un simple macérât huileux. Ainsi, suivant l'invention on associe les avantages d'une huile végétale et de molécules hydrophiles (flavonoïdes, composés phénoliques). Enfin, le véhicule huileux évite de devoir ajouter un conservateur à la composition, celui-ci permettant en effet de stabiliser dans le temps les propriétés antioxydantes de l'extrait lipophile d'hémérocalle et donc l'efficacité des compositions cosmétiques la renfermant.

Selon une forme de réalisation préférée de l'invention, le véhicule huileux comprend un mélange d'huile d'avocat et d'huile de rosier muscat. Dans ce cas, le rapport massique huile d'avocat/huile de rosier muscat au sein de la composition huileuse varie de préférence de 4/1 à 1/1 environ, et encore plus préférentiellement de 2/1 à 1/1.

Au sein de la composition huileuse utilisable selon l'invention, la teneur en extrait lipophile d'hémérocalle (molécules lipophiles extraites de la plante hémérocalle, soit principalement des caroténoïdes et du β-carotène) peut varier de 5 à 20 ppm, et plus préférentiellement, de 15 à 20 ppm.

Les compositions utilisables selon l'invention peuvent contenir, outre la composition huileuse conforme à l'invention, un ou plusieurs actifs secondaires renforçant ou complétant avantageusement son activité, et compatibles, c'est-à-dire non susceptibles de réagir les uns avec les autres ou de masquer ou limiter ses effets.

Plus particulièrement, les actifs secondaires peuvent être choisis parmi un agent anti-âge, ou parmi l'acétyl tetrapeptide-5 (CAS N° 820959-17-9), un extrait de plancton, un extrait de maca qui a un effet sur la prévention de dérèglements liés à une déficience de la microcirculation cutanée et au stress oxydatif qui peut en résulter, un extrait de pétales de coquelicot agissant sur la nutrition cellulaire, une combinaison d'extraits d'anchuse, de coquelicot et de passiflore qui, en association, ont un effet dans la prévention des signes du vieillissement cutané, un extrait de graines de mimosa, un extrait de pétales de souci, les cellules de cacao, la vitamine C, la vitamine E et l'hexylrésorcinol.

Les compositions utilisables selon l'invention peuvent en particulier contenir en outre un ou plusieurs agents dépigmentants, notamment d'origine végétale, qui peuvent avoir un effet complémentaire et parfaire la luminosité du teint et l'uniformisation du teint. Parmi de tels agents dépigmentants, on peut par exemple citer les extraits de murier blanc, les extraits de lys des mers, la vitamine C, les cellules de cacao, les pétales de capucine, les extraits d'algues, ...

L'agent anti-âge peut être par exemple un lipide tel que le géranyl géranyl isopropanol (Juvinity®) qui réduit la formation des peroxydes intracellulaires et retarde ainsi le vieillissement cellulaire.

Les compositions cosmétiques utilisables selon la présente invention possèdent une excellente tolérance cutanée, notamment en raison du film lipidique résultant du support huileux (huiles végétales, en particulier huile d'avocat et huile de rosier muscat), elles ne présentent aucune phototoxicité et leur application sur la peau, pour des périodes de temps prolongées, n'implique aucun effet systémique.

Les compositions utilisables selon la présente invention peuvent se présenter sous les formes galéniques classiquement utilisées pour une application topique, c'est-à-dire sous forme de gel, d'émulsion (en particulier crème ou lait), d'émulsion biphasique huile-dans-eau ou eau-dans-huile, d'huile corporelle, de masque, de pommade, d'onguent, de lotion, de solution concentrée, de nanocapsules, de liposomes, lesdites formes galéniques contenant en outre des excipients et supports usuels et acceptables sur le plan cosmétologique. Elles sont utilisées de préférence sous forme de crèmes, de sérums, de lotion ou de gel.

Ces formes d'administration par voie topique sont préparées par les techniques usuelles, et par exemple, dans le cas d'une crème, par dispersion d'une phase grasse dans une phase aqueuse pour obtenir une émulsion huile-dans-eau, ou inversement pour préparer une émulsion eau-dans-huile. Dans le cas de crèmes, on peut utiliser des émulsions à structure lamellaire obtenues avec des lécithines hydrogénées ou des sucro-esters, contenant peu de produits éthoxylés ou n'en contenant pas du tout.

Les compositions cosmétiques utilisables suivant l'invention peuvent aussi prendre la forme de lotion ou solution dans laquelle les extraits sont sous forme encapsulée dans des microsphères. Les microsphères suivant l'invention peuvent par exemple être constituées de corps gras, d'agar et d'eau. La composition huileuse utilisable conformément à l'invention peut être incorporée dans des vecteurs de type liposomes, glycosphères, dans des chylomicrons, des macro-, micro-, nano-particules ainsi que les macro-, micro- et nanocapsules et aussi être adsorbés sur des polymères organiques poudreux, les talcs, bentonites et autres supports minéraux.

Ces émulsions jouissent d'une bonne stabilité et peuvent être conservées pendant le temps nécessaire pour l'utilisation à des températures comprises entre 0 et 50 °C sans qu'il y ait sédimentation des constituants ou séparation des phases.

Les compositions topiques utilisables selon l'invention peuvent comprendre des excipients appropriés pour une application topique externe, en particulier des excipients acceptables sur le plan cosmétologique. Ces excipients appropriés pour la formulation sont bien connus de l'homme du métier et comprennent en particulier des agents de pénétration tels que le phytantriol, l'octyl dodécanol et l'escine ; les épaississants tels que les gommes naturelles et les polymères de synthèse ; les tensioactifs ; les émollients tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsionnants tels que des dérivés de polyglycérol ; les conservateurs tels que le phénoxyéthanol et l'acide déhydroacétique ; les colorants ; les parfums ; etc...

Comme autres ingrédients utilisables dans les compositions de l'invention, on peut citer les agents hydratants tels que la glycérine, le butylène glycol, le sel de sodium de l'acide pyrrolidone carboxylique (sodium PCA), ainsi que des associations de dérivés glucosiques à effet hydratant et restructurant comme le produit Aquaxyl® (xylitylglucoside et anhydroxylytol et xylitol), et également les vitamines antioxydantes telles que la vitamine E, par exemple l'acétate de tocophérol ou le tocotriénol, la vitamine C, les polyphénols naturels.

On peut aussi ajouter à la composition des glucides choisis principalement parmi le glucose, le glycogène et le tréhalose, ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que d'une manière générale toute association avec un céramide.

On peut également ajouter à la composition des agents de protection contre les rayons ultraviolets, et par exemple des filtres solaires UV-A et UV-B hydrophiles ou lipophiles, ou des pigments formant écran anti-ultraviolet.

Les filtres solaires peuvent être choisis par exemple parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-β,β-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butyl-méthoxy dibenzoylméthane (Parsol 1789®), et le 4-méthoxy-dibenzoylméthane.

Les pigments peuvent être choisis par exemple parmi le dioxyde de titane, l'oxyde de zinc et l'oxyde de zirconium.

L'utilisation cosmétique de la composition cosmétique conforme à l'invention comprend tous les soins de la peau, en particulier les soins du visage, y compris les produits solaires, protecteurs et bronzants, les produits anti-âge, anti-séborrhéiques, toniques, les produits assurant l'amélioration de l'aspect de la peau y compris le traitement acnéique et les rougeurs cutanées.

Les exemples suivants illustrent l'invention plus en détail sans en limiter la portée. Dans tous les exemples de compositions qui suivent, les parties sont exprimées en masse, sauf indication contraire.

### EXEMPLES

### EXEMPLE 1 : Préparation d'une composition huileuse comprenant un extrait lipophile d'hémérocalle

Une composition huileuse à base d'extrait lipophile *d'Hemerocallis fulva* a été préparée comme indiqué ci-après.

On a utilisé l'ensemble des parties aériennes de la plante entière, préalablement séchée, puis broyée à froid. La technique du séchage/broyage à froid est préférable car elle permet de préserver les anti-oxydants présents dans la plante. Suivant cette technique, les plantes sont soumises à un premier broyage, puis à une déshydratation à température ambiante à l'abri de la lumière, de manière à ramener la teneur en eau vers 10%, et broyées à froid, à une température de -80°C, au moyen d'un broyeur à couteaux.

La teneur en eau était inférieure à 12 % après broyage et séchage à froid, ce pourcentage pouvant notamment varier de 8,5 à 12 % environ.

20 kg d'hémérocalle ainsi séchée et broyée, sous forme de poudre fine et homogène, ont été mélangés à 60 kg d'huile d'avocat et 20 kg d'huile de rosier muscat (rapport massique huile d'avocat/huile de rosier muscat = 3/1). Le rapport massique plante / huile était de 0,20.

Le mélange a ensuite été chauffé rapidement par micro-ondes à une puissance de 10000 à 30000 Watts/ kg de mélange, pendant une durée inférieure à 10 minutes, sous flux d'azote, la température maximale atteinte étant comprise entre 80 et 200°C.

Le mélange a ensuite été traité par ultrasons (100 kHz environ), sous azote et sous agitation, pendant 10 à 20 minutes environ. Le mélange d'huiles vectrices contenant l'extrait lipophile *d'Hemerocallis fulva* a été séparé par centrifugation.

### EXEMPLE 2 : Mise en évidence de l'absence de cytotoxicité de la composition huileuse conforme à l'invention

Dans cet exemple on a testé la cytotoxicité éventuelle de la composition huileuse conforme à l'invention, telle qu'obtenue par le procédé de l'Exemple 1.

Le test sur la viabilité d'épidermes humains reconstruits utilisé est celui de la réduction au bleu de Formazan (test MTT). L'essai a été réalisé *in vitro* sur épidermes humains reconstruits (« *Reconstructed Human Epidermis* », RHE) modèle RHEps, de surface 0,5 cm², vendus par la société SkinEthic ®.

Le mélange d'huiles vectrices (huile d'avocat/huile de rosier muscat) dans le rapport massique 3/1 tel qu'utilisé pour l'extraction réalisée à l'exemple 1 a été testé pur (100 %), en application topique sur les épidermes.

La composition huileuse telle que préparée à l'exemple 1 a été testée après dilution à 0,5% (v/v), 1% (v/v), 1,5% (v/v) et 3% (v/v) dans de l'huile de paraffine. Chacune des compositions huileuses ainsi diluées a été appliquée à raison de 5 mg/cm², sur le stratum corneum des épidermes.

Les mesures de viabilité tissulaires par test MTT ont été effectués après 24 heures d'incubation à 37°C en atmosphère humide air-CO₂ (35%/5%), un épiderme non traité servant de témoin.

Les résultats sont reportés dans le tableau I ci-après.

**TABLEAU I**

| **Substance** | **Densité optique à 540 nm** | **Viabilité (%)** |
|---|---|---|
| Aucune (Témoin) | 1,058 ± 0,007 | 100 |
| Huiles vectrices pures | 1,058 ± 0,010 | 100 |
| Composition huileuse à 0,5 % | 1,048 ± 0,013 | 99 |
| Composition huileuse à 1 % | 1,057 ± 0,007 | 100 |
| Composition huileuse à 1,5 % | 1,050 ± 0,006 | 99 |
| Composition huileuse à 3,0 % | 1,031 ± 0,007 | 97 |

Ces résultats montrent qu'aucune diminution significative de la viabilité cellulaire n'est enregistrée après traitement topique avec la composition huileuse conforme à la présente invention dans la gamme de concentrations testées ; ceci comparativement au mélange des deux huiles vectrices testées dans les mêmes conditions qui ne présente lui non plus, aucun effet toxique vis-à-vis des tissus cutanés.

### EXEMPLE 3 : Mise en évidence des propriétés de la composition huileuse selon l'invention sur l'éclat du teint

Dans cet exemple, on a évalué les effets de deux compositions sous forme de crème sur l'amélioration du teint, lesdites compositions comprenant des quantités différentes de la composition huileuse selon l'invention, à savoir :
- Composition huileuse telle que préparée à l'exemple 1, formulée à 1 % en masse dans une crème pour le visage (ci-après dénommée « Crème à 1 % ») ;
- Composition huileuse telle que préparée à l'exemple 1, formulée à 4 % en masse dans une crème pour le visage (ci-après dénommée « Crème à 4 % »).

La formulation de la crème à 1 % était la suivante :
- Eau 92,9 %
- Huile de tournesol 4 %
- Copolymère d'ammonium acryloyldiméthyltaurate et de vinylpyrrolidone vendu sous la dénomination commerciale Aristoflex® AVC par Clariant 2 %
- Conservateur 1%
- Composition huileuse de l'exemple 1 1 %

La formulation de la crème à 4 % était en tout point identique à celle de la crème à 1 % ci-dessus sauf qu'elle contenait 4 % en masse de la composition huileuse de l'exemple et seulement 89,9 % en masse d'eau.

### 3.1. Principe du test

Le principe du test est basé sur l'évaluation de l'activité protectrice des crèmes vis-à-vis de la formation de dityrosine. La tyrosine est l'une des cibles majeures de l'oxydation des protéines. La dityrosine est un dimère de la tyrosine pouvant se former sous l'action de dérivés réactifs de l'oxygène, de radiations ultraviolets ou encore d'enzymes telles que les peroxydases. La dityrosine est un marqueur spécifique de la lipofuscine.

### 3.2. Protocole

L'étude a été réalisée sur des explants de peau humains d'un diamètre de 10 mm préparés à partir d'une plastie abdominale d'une femme âgée de 53 ans.

27 explants ont été prélevés. A réception, les explants ont été transférés dans des plaques de 12 puits contenant du milieu de culture pour épiderme vendu par la société SkinEthic (0,5 ml/puits) et placés à l'incubateur à 37°C, dans une atmosphère à 5% de CO₂ saturée d'humidité, pendant 24 heures avant de procéder au traitement des tissus.

Les explants ont ensuite subi différents traitements, chaque traitement ayant été réalisé en triplicate :
**Lot TEMOIN T0** : Aucun traitement, prélèvement à 0 jours ;
**Lot TEMOIN J9** : Aucun traitement, prélèvement à 9 jours ;
**Lot Crème à 1 %** : traitement avec la crème à 1 %, prélèvement à 9 jours ;
**Lot Crème à 4 %** : traitement avec la crème à 4 %, prélèvement à 9 jours ;
**Lot MG :** Induction de la glycation par le méthylglyoxal, aucun traitement, prélèvement à 9 jours ;
**Lot Crème à 1 % + MG** : Induction de la glycation par le méthylglyoxal, traitement avec la crème à 1 %, prélèvement à 9 jours ;
**Lot Crème à 4 % + MG :** Induction de la glycation par le méthylglyoxal, traitement avec la crème à 4 %, prélèvement à 9 jours.

### Application des produits :

Aux jours J0, J2, J3 et J6, les produits (crème à 1% ou crème à 4 %) ont été appliqués sur les explants à raison de 2 mg de crème par explant. Les explants des lots TEMOIN T0 et TEMOIN J9 n'ont reçu aucun traitement. Les milieux de culture ont été renouvelés aux jours J3 et J6.

### Induction de la glycation :

Aux jours J3 et J6, 18 µl d'une solution de méthylglyoxal à 55 mM dans l'eau stérile (cette solution a été préparée à partir d'une solution commerciale de méthylglyoxal Sigma M0252 à 40%) ont été ajoutés aux puits contenant les explants des lots **MG** ; **Crème à 1 % + MG** et **Crème à 4 % + MG.** La concentration finale en MG dans chacun des puits était de 500 µM.

### Prélèvements des explants :

A J0, les explants du lot TEMOIN T0 ont été prélevés et coupés en deux. Une moitié a été fixée par du formol tamponné pendant 24 heures et l'autre moitié a été congelée à - 80°C.

A J9, les 3 explants de chacun des lots ont été prélevés et traités de la même façon.

### Traitement histologique des explants

Après 24 heures de fixation dans le formol tamponné, les prélèvements ont été déshydratés et imprégnés en paraffine à l'aide d'un automate de déshydratation Leica ® TP 1010.

Les prélèvements ont ensuite été mis en bloc de paraffine à l'aide d'une station d'enrobage Leica ® EG 1160.

Des coupes de 5 µm ont été réalisées à l'aide d'un microtome type Minot, Leica ® RM 2125, puis fixées et montées sur des lames de verre histologiques pour les analyses de morphologie générale.

Les prises de vue ont été réalisées avec une caméra Olympus DP72 ® et le logiciel d'archivage de données Cell-D.

### Immuno-marquage :

Comme indiqué en préambule de cet exemple, le marquage de la lipofuscine a été réalisé *via* la dityrosine, selon le protocole suivant :
Les coupes histologiques ont d'abord été déparaffinées dans des bains de xylène et rincées à l'alcool absolu.

Le marquage de la dityrosine a été réalisé avec un anticorps monoclonal de souris anti-dityrosine (clone V1C3, société Cosmo Bio, référence *NNS-MDT-020P-EX)* au 1/25^{ème} pendant 1 heure à température ambiante avec un système amplificateur vendu sous la dénomination commerciale VECTASTAIN® Elite ABC Reagent, R.T.U. (« *Ready-to-Use* ») basé sur l'interaction versatile biotine/avidine et révélé avec un kit à la peroxydase vendu sous la dénomination Vector® VIP SUBSTRATE KIT FOR PEROXIDASE, référence SK-4600 par la société Vector Laboratories.

Les lames ont été observées avec une caméra Olympus DP72 ® et le logiciel Cell-D.

Après immuno-marquage, le pigment de lipofuscine apparait en jaune sur les explants de peau colorés et en gris foncé sur les photos prises en noir et blanc.

### Test quantitatif

Les études des coupes histologiques ont été complétées par la réalisation d'un test semi-quantitatif sur les lots TEMOIN T0, TEMOIN J9, Crème à 1%, MG et Crème à 1 % + MG afin de déterminer la surface occupée par la dityrosine dans l'épiderme.

La surface occupée par la dityrosine au niveau de l'épiderme a été mesurée par analyse d'image grâce au logiciel QWin® de la société Leica. Ce logiciel permet de mesurer différents paramètres morphologiques tels que la surface, la circularité (pour les fibroblastes, plus ou moins ronds ou fusiformes) ou encore le nombre de fourches d'une cellule (témoin de la dendricité, dans ce cas dendricité du fibroblaste, et témoignant de la bonne santé et activité de la cellule).

8 à 12 photos par lot ont été analysées.

### 3.3. Résultats

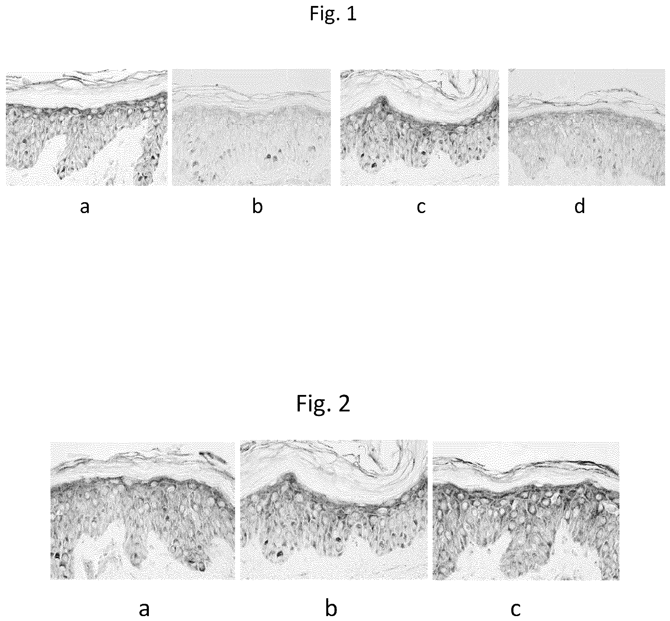

Les photos des coupes des explants correspondant à chacun des lots sont données sur les figures 1 et 2 annexées :
- Figure la : coupe d'un explant du lot TEMOIN J9 ;
- Figure 1b : coupe d'un explant du lot Crème à 1 % ;
- Figure 1c : coupe d'un explant du lot MG ;
- Figure 1d : coupe d'un explant du lot Crème à 1 % + MG ;
- Figure 2a : coupe d'un explant du lot Crème à 4 % ;
- Figure 2b : coupe d'un explant du lot MG ;
- Figures 2c : coupe d'un explant du lot Crème à 4 % + MG.

En absence de traitement (photo de la figure la), on remarque une coloration grisâtre générale sur tout l'épiderme ainsi que qu'une concentration plus importante dans le *stratum granulosum* traduisant de la présence de dityrosine.

On constate que l'application de la crème formulée avec la composition huileuse à 1% induit une diminution assez nette de la formation de dityrosine au niveau du *stratum granulosum* et inhibe totalement la formation de dityrosine sur le reste de l'épiderme (photo de la figure 1b).

Par contre, l'application de la crème formulée avec la composition huileuse à 4 % n'induit pas de diminution de dityrosine. Il n'y a donc pas de diminution significative de lipofuscine quand on augmente la concentration en composition huileuse (Photo de la figure 2a).

Après incorporation du MG dans le milieu de culture et comparativement au lot témoin à J9 **(TEMOIN J9),** la formation de dityrosine est légèrement augmentée au niveau du *stratum granulosum* et assez nettement sur le reste de l'épiderme. Le stress au MG induit donc une production de dityrosine supérieure à celle de l'épiderme non traité par MG (photo de la figure 1c et 2b).

L'application de la crème à 1% après que l'explant ait été mis en contact avec MG (stress glyquant) induit une très nette diminution de la formation de dityrosine au niveau du *stratum granulosum* et inhibe totalement la formation de dityrosine sur le reste de l'épiderme (Photo de la figure 1d).

Par contre, l'application de la crème à 4% après soumission de l'explant à un stress glyquant, n'induit aucune diminution de dityrosine contrairement à la crème à 1% (Photo de la figure 2c). On note même une augmentation modérée de la quantité de dityrosine au niveau du *stratum granulosum* et une augmentation très légère sur le reste de l'épiderme.

Les résultats du test semi-quantitatif de la surface occupée par la dityrosine sont donnés dans le tableau II ci-après :

**TABLEAU II**

| **Lot** | **Surface occupée par la dityrosine (en %)** | |
|---|---|---|
| | **Moyenne** | **Ecart type** |
| **TEMOIN T0** | 21,6 | 8,4 |
| **TEMOIN J9** | 7,6 | 5,8 |
| **Crème à 1 %** | 0,5 | 0,7 |
| **MG** | 13,8 | 3,7 |
| **Crème à 1 % + MG** | 0,6 | 0,6 |

Il ressort de ces résultats qu'à J0 (TEMOIN T0) la dityrosine occupe 21,6% de la surface. Au bout de 9 jours (TEMOIN J9), la formation de dityrosine est diminuée de 65 % comparativement au lot TEMOIN T0.

Après application de la crème à 1 % et au bout de 9 jours de traitement (Lot Crème à 1%), on observe que l'application du produit entraine une diminution significative de 93 % de la formation de dityrosine dans l'épiderme.

Après incorporation du MG et en l'absence de traitement (Lot MG), la formation de dityrosine est augmentée significativement de 82 %.

Comparativement au lot traité avec du MG seul (Lot MG), et après 9 jours de traitement, l'application de la crème formulée à 1 % en masse de composition huileuse après stress glyquant (Lot Crème à 1 % + MG) induit une diminution significative de 96 % de la formation de dityrosine dans l'épiderme.

### Conclusion

L'ensemble de ces résultats met en évidence que crème contenant 1% de composition huileuse permet une nette diminution de la quantité de lipofuscine à la fois dans le *stratum granulosum* et dans le reste de l'épiderme contrairement à la crème contenant 4% de composition huileuse pour laquelle aucune activité protectrice n'a été observée.

On peut donc en déduire que la composition huileuse utilisée à 1% en masse dans une formulation a une action significative sur la diminution de ce pigment produit et accumulé dans les tissus avec l'âge aussi bien dans des conditions normales de vieillissement que quand la peau est soumise à un stress nettement plus important (stress glyquant).

Au vu de la composition de la composition huileuse utilisée conformément à l'invention, on peut avancer que son efficacité est très certainement liée à la quantité très importante de molécules antioxydantes telles que des acides chlorogéniques (mesure de 416 ppm) permettant de diminuer les protéines oxydées.

Par contre, quand la quantité en composition huileuse est trop importante (4 % en masse) on obtient un effet inverse. Si on considère qu'une activité est souvent représentée par une courbe en « cloche », on peut penser dans le cas du marqueur « dityrosine » que l'on obtient le maximum d'efficacité entre 1 et 2% et qu'au-delà l'efficacité diminue à nouveau.

Les résultats chiffrés correspondant à la crème à 1 % confirment bien que la composition huileuse contenant l'extrait lipophile d'hémérocalle utilisée conformément à l'invention a une action très efficace sur la formation de dityrosine aussi bien sur une peau non stressée (93% de réduction de formation de dityrosine) que sur une peau exposée à un agent glyquant (96 % de réduction de formation de dityrosine).

Ces essais démontrent que la composition huileuse utilisée conformément à l'invention et comprenant un extrait lipophile d'hémérocalle a une action très significative sur la formation de lipofuscine aussi bien pour une peau jeune que pour une peau plus mature et donc par voie de conséquence sur l'éclat du teint.

### EXEMPLE 4 : Etude clinique

Une étude clinique a été réalisée de façon strictement confidentielle sur un panel de 270 volontaires afin d'assoir l'allégation « amélioration de l'éclat du teint » sur une période de 28 jours. Il est à noter que sur les 270 volontaires, seuls 256 ont effectué l'étude dans sa durée totale. La composition des formules n'était pas mentionnée sur les emballages et n'a pas été communiquée aux membres du panel.

Il a été réalisé sur des produits de soin appartenant à la gamme vendue sous la dénomination commerciale Merveillance® par la société Nuxe, dans lesquels a été ajouté 1 % en masse de composition huileuse telle que préparée ci-dessus à l'exemple 1.

Les références testées de la Gamme Merveillance® sont les suivantes :
- Crème Peaux Sèches à Très Sèches (PS-TS),
- Crème Peaux Normales à Sèches (PN-PS),
- Crème Peaux Normales à Mixtes (PN-PM),
- Crème de nuit Peaux Normales à Sèches (Nuit-PN-PS),
- Contour des yeux (CY),
- Sérum.

Chacun de ces produits commerciaux a été modifié en y ajoutant 1 % en masse d'une composition huileuse telle que préparée ci-dessus à l'exemple.

Pour cette étude, les volontaires ont été divisés en 4 groupes :
- Groupe 1 (G1) : Sérum + CY + PN-PS,
- Groupe 2 (G2) : Sérum + CY + Nuit-PN-PS,
- Groupe 3 (G3) : Sérum + CY + PS-TS,
- Groupe 4 (G4) : Sérum + CY + PN-PM.

Les produits ont été appliqués selon les recommandations suivantes :
Sérum + contour des yeux : une application le matin ;
Les crèmes (PS-TS ; PN-PS ; PN-PM) : une application le matin et une application le soir.

Pendant la période du test, les membres du panel n'ont appliqué aucun autre produit cosmétique que ceux testés sur leur peau.

Le tableau VI ci-après regroupe les traitements reçus en fonction du nombre de volontaires :

**TABLEAU III**

| **Nombre de volontaires** | **Sérum** | **CY** | **PN-PS** | **Nuit-PN-PS** | **PS-TS** | **PN-PM** |
|---|---|---|---|---|---|---|
| **à J0** | 270 | 270 | 68 | 68 | 67 | 67 |
| **à J28** | 256 | 261 | 66 | 63 | 63 | 65 |

Il a été demandé à chaque volontaire, à J0 et à J28, de répondre par « oui » ou par « non » à la question suivante : votre teint vous semble-t-il plus éclatant (plus lumineux) ?

Les réponses des membres du panel (en %), en fonction des groupes, sont reportées dans le tableau VII ci-après :

**TABLEAU IV**

| **Temps** | **G1** | **G2** | **G3** | **G4** |
|---|---|---|---|---|
| **à J0** | 59 % | 72 % | 63 % | 66 % |
| **à J28** | 85 % | 91 % | 78 % | 80 % |

Au vu de ces résultats, le nombre de testeurs étant conséquent, on peut conclure que l'application des produits comprenant 1 % en masse de composition huileuse conforme à l'invention conduit bien à gain de luminosité, c'est-à-dire à une amélioration de l'éclat du teint.

### EXEMPLE 5 : Crème peau sèche éclat du teint

Par les techniques usuelles, on a préparé une crème pour peux sèches destinée à améliorer l'éclat du teint et ayant la composition massique suivante :
- EDTA tetrasodique 0,1 g
- Caféine 0,2 g
- Glycérine 3,0 g
- Polymère réticulé d'acrylate/C₁₀₋₃₀ alkyle
   acrylate vendu sous la dénomination commerciale Carbopol® Ultrez 21 par Gattefossé 1,0 g
- Mélange d'alcool arachidylique, d'alcool
   Behénique et de glucoside d'arachidyle vendu sous la dénomination Montanov® 202 par SEPPIC 4,0 g
- Huile de noisette 6,0 g
- Beurre de karité 2,0 g
- Oléate de décyle 3,0 g
- Tocophérol 0,7 g
- Composition huileuse de l'exemple 1 2,0 g
- Système de conservation 0,8 g
- NaOH 0,08 g
- Parfum 0,5 g
- Eau qsp 100,0 g

Cette crème peut être utilisée une à deux fois par jour par application sur les zones de la peau à traiter (visage).

### EXEMPLE 6 : Sérum éclat du teint

Par les techniques usuelles, on a préparé un sérum destiné à améliorer l'éclat du teint ayant la composition massique suivante :
- EDTA tetrasodique 0,1 g
- Extrait de plancton vendu sous la dénomination
   EPS Seafill par la société CODIF 3,0 g
- Glycérine 3,0 g
- Carbomer vendu sous la dénomination Carbopol®
   Ultrez 10 par la société Gattefossé 0,2 g
- Glutamate de stéaryle sodique 0,5 g
- Huile de rosier muscat 2,0 g
- Oléate de décyle 1,0 g
- Copolymère réticulé à base de polydiméthysiloxane vendu sous la dénomination commerciale DC9041 par la société Dow Corning 1,0 g
- Tocophérol 0,7 g
- Composition huileuse de l'exemple 1 5,0 g
- Hyaluronate de sodium 0,3 g
- Soude 0,15 g
- Système de conservation 0,8 g
- Parfum 0,5 g
- Eau qsp 100,0 g

Ce sérum peut être utilisé une à deux fois par jour par application sur les zones de la peau à traiter (visage).

## Revendications

1. Utilisation, à titre d'ingrédient actif, d'une composition huileuse à base d'un extrait lipophile *d'Hemerocallis fulva* et d'un véhicule huileux comprenant au moins une huile végétale, pour la préparation d'une composition cosmétique à usage topique destinée à améliorer l'éclat du teint et/ou à uniformiser le teint, ladite composition huileuse représentant de 0,01 à 2 % en masse par rapport à la masse totale de ladite composition cosmétique.

2. Utilisation selon la revendication 1, **caractérisée en ce que** la composition huileuse représente de 0,1 à 2 % en masse par rapport à la masse totale de la composition cosmétique.

3. Utilisation selon la revendication 1, **caractérisée en ce que** la composition huileuse représente 1 % en masse par rapport à la masse totale de la composition cosmétique.

4. Utilisation selon l'une quelconque des revendications précédentes, **caractérisée** en ledit extrait est obtenu à partie des parties aériennes *d'Hemerocallis fulva.*

5. Utilisation selon l'une quelconque des revendications précédentes, **caractérisée en ce que** ledit extrait est obtenu par extraction à l'abri de l'air.

6. Utilisation selon l'une quelconque des revendications précédentes, **caractérisée en ce que** le véhicule huileux comprend au moins une huile végétale naturelle choisie parmi l'huile d'avocat, l'huile de rosier muscat, l'huile de noisette, l'huile de colza, l'huile de macadamia, l'huile d'argan, l'huile de tournesol, l'huile de cameline, l'huile de bourrache, l'huile d'amande douce, l'huile de carthame, l'huile de calophyllum et leurs mélanges.

7. Utilisation selon la revendication 6, **caractérisée en ce que** l'huile végétale naturelle est choisie parmi l'huile d'avocat, l'huile de rosier muscat et leurs mélanges.

8. Utilisation selon la revendication 7, **caractérisée en ce que** le véhicule huileux comprend un mélange d'huile d'avocat et d'huile de rosier muscat.

9. Utilisation selon la revendication 8, **caractérisée en ce que** le rapport massique huile d'avocat/huile de rosier muscat au sein de la composition huileuse varie de 4/1 à 1/1.

10. Utilisation selon l'une quelconque des revendications précédentes, **caractérisée en ce que** la teneur en extrait lipophile d'hémérocalle varie de 5 à 20 ppm.

11. Utilisation selon l'une quelconque des revendications précédentes, **caractérisée** en ce la composition cosmétique contient en outre un ou plusieurs actifs secondaires choisis parmi un agent anti-âge, l'acétyl tetrapeptide-5, un extrait de plancton, un extrait de maca, un extrait de pétales de coquelicot, une combinaison d'extraits d'anchuse, de coquelicot et de passiflore, un extrait de graines de mimosa, un extrait de pétales de souci, les cellules de cacao, la vitamine C, la vitamine E et l'hexylrésorcinol.

12. Utilisation selon l'une quelconque des revendications précédente, **caractérisée en ce que** la composition cosmétique se présente sous forme de gel, d'émulsion, d'émulsion biphasique huile-dans-eau ou eau-dans-huile, d'huile corporelle, de masque, de pommade, d'onguent, de lotion, de solution concentrée, de nanocapsules ou de liposomes.

13. Utilisation selon l'une quelconque des revendications précédentes, **caractérisée en ce que** la composition cosmétique comprend en outre des agents de protection contre les rayons ultraviolets ou des pigments formant écran anti-ultraviolet.

14. Procédé cosmétique non thérapeutique pour améliorer l'éclat du teint et/ou uniformiser le teint, consistant à appliquer sur les zones de la peau concernées au moins une composition cosmétique topique contenant de 0,01 à 2 % en masse d'une composition huileuse à base d'un extrait lipophile *d'Hemerocallis fulva* et d'un véhicule huileux comprenant au moins une huile végétale.

15. Utilisation non thérapeutique d'une composition cosmétique à application topique comprenant de 0,01 à 2 % en masse d'une composition huileuse à base d'un extrait lipophile *d'Hemerocallis fulva* et d'un véhicule huileux comprenant au moins une huile végétale, pour améliorer l'éclat du teint et/ou uniformiser le teint.

## Patentansprüche

1. Verwendung als aktiver Inhaltsstoff einer öligen Zusammensetzung auf der Basis eines lipophilen *Hemerocallis fulva*-Extrakts und eines öligen Vehikels, umfassend mindestens ein Pflanzenöl, für die Zubereitung einer kosmetischen Zusammensetzung für topische Anwendung, die dazu bestimmt ist, die Ausstrahlung des Teints zu verbessern und/oder den Teint ebenmäßig zu machen, wobei die ölige Zusammensetzung 0,01 bis 2 Ma% in Bezug zur Gesamtmasse der kosmetischen Zusammensetzung darstellt.

2. Verwendung nach Anspruch 1, **dadurch gekennzeichnet, dass** die ölige Zusammensetzung 0,1 bis 2 Ma% in Bezug zur Gesamtmasse der kosmetischen Zusammensetzung darstellt.

3. Verwendung nach Anspruch 1, **dadurch gekennzeichnet, dass** die ölige Zusammensetzung 1 Ma% in Bezug zur Gesamtmasse der kosmetischen Zusammensetzung darstellt.

4. Verwendung nach einem der vorangehenden Ansprüche, **dadurch gekennzeichnet, dass** der Extrakt aus den oberirdischen Teilen von *Hemerocallis fulva* gewonnen wird.

5. Verwendung nach einem der vorangehenden Ansprüche, **dadurch gekennzeichnet, dass** der Extrakt durch Extraktion geschützt vor Luft gewonnen wird.

6. Verwendung nach einem der vorangehenden Ansprüche, **dadurch gekennzeichnet, dass** das ölige Vehikel mindestens ein natürliches Pflanzenöl, ausgewählt aus dem Avocadoöl, dem Wildrosenöl, dem Haselnussöl, dem Rapsöl, dem Macadamiaöl, dem Arganöl, dem Sonnenblumenöl, dem Leindotteröl, dem Borretschöl, dem Mandelöl, dem Färberdistelöl, dem Calophyllumöl und ihren Gemischen, ist.

7. Verwendung nach Anspruch 6, **dadurch gekennzeichnet, dass** das natürliche Pflanzenöl aus dem Avocadoöl, dem Wildrosenöl und ihren Gemischen ausgewählt ist.

8. Verwendung nach Anspruch 7, **dadurch gekennzeichnet, dass** das ölige Vehikel ein Gemisch aus Avocadoöl und Wildrosenöl umfasst.

9. Verwendung nach Anspruch 8, **dadurch gekennzeichnet, dass** das Massenverhältnis Avocadoöl/Wildrosenöl innerhalb der öligen Zusammensetzung von 4/1 bis 1/1 schwankt.

10. Verwendung nach einem der vorangehenden Ansprüche, **dadurch gekennzeichnet, dass** der Gehalt an liphophilem Hemerocallisextrakt von 5 bis 20 ppm schwankt.

11. Verwendung nach einem der vorangehenden Ansprüche, **dadurch gekennzeichnet, dass** die kosmetische Zusammensetzung ferner einen oder mehrere sekundäre Wirkstoffe enthält, ausgewählt aus einem Anti-Age-Mittel, dem Acetyltetrapeptid-5, einem Planktonextrakt, einem Macaextrakt, einem Extrakt der Blütenblätter des Klatschmohns, einer Kombination von Extrakten aus Ochsenzunge, Klatschmohn und Passionsblume, einem Extrakt aus Mimosensamen, einem Extrakt aus den Blütenblättern der Ringelblume, den Kakaozellen, dem Vitamin C, dem Vitamin E und dem Hexylresorcinol.

12. Verwendung nach einem der vorangehenden Ansprüche, **dadurch gekennzeichnet, dass** die kosmetische Zusammensetzung in Form von Gel, Emulsion, Zweiphasen-Emulsion Öl-in-Wasser oder Wasser-in-ÖI, Körperöl, Maske, Pomade, Salbe, Lotion, von konzentrierter Lösung, Nanokapseln oder Liposomen vorliegt.

13. Verwendung nach einem der vorangehenden Ansprüche, **dadurch gekennzeichnet, dass** die kosmetische Zusammensetzung comprend ferner Schutzmittel gegen die ultravioletten Strahlen oder Pigment, die einen Schutzschirm vor UV bilden, umfasst.

14. Nicht therapeutisches kosmetisches Verfahren zur Verbesserung der Ausstrahlung des Teints und/oder um den Teint ebenmäßig zu machen, das darin besteht, auf die betroffenen Zonen der Haut mindestens eine topische kosmetische Zusammensetzung aufzutragen, die 0,01 bis 2 Ma% einer öligen Zusammensetzung auf der Basis eines lipophilen *Hemerocallis fulva*-Extrakts und eines öligen Vehikels, umfassend mindestens ein Pflanzenöl, enthält.

15. Nicht therapeutische Verwendung einer kosmetischen Zusammensetzung mit topischer Anwendung, umfassend 0,01 bis 2 Ma% einer öligen Zusammensetzung auf der Basis eines lipophilen *Hemerocallis fulva*-Extrakts und eines öligen Vehikels, umfassend mindestens ein Pflanzenöl, zur Verbesserung der Ausstrahlung des Teints und/oder um den Teint ebenmäßig zu machen.

## Claims

1. Use, as active ingredient, of an oily composition containing a lipophilic extract of *Hemerocallis fulva* and an oily vehicle comprising at least one vegetable oil, to prepare a cosmetic composition for topical use intended to improve skin radiance and/or to uniformize the complexion, said oily composition representing 0.01 to 2 % by weight relative to the total weight of said cosmetic composition.

2. The use according to claim 1, **characterized in that** the oily composition represents 0.1 to 2 % by weight relative to the total weight of the cosmetic composition.

3. The use according to claim 1, **characterized in that** the oily composition represents 1 % by weight relative to the total weight of the cosmetic composition.

4. The use according to any of the preceding claims, **characterized in that** said extract is obtained from the above-ground parts of *Hemerocallis fulva.*

5. The use according to any of the preceding claims, **characterized in that** said extract is obtained by airtight extraction.

6. The use according to any of the preceding claims, **characterized in that** the oily vehicle comprises at least one natural vegetable oil selected from among avocado oil, rose hip oil, hazelnut oil, rapeseed oil, macadamia oil, argan oil, sunflower seed oil, camelina oil, borage oil, sweet almond oil, safflower oil, tamanu oil and mixtures thereof.

7. The use according to claim 6, **characterized in that** the natural vegetable oil is selected from among avocado oil, rose hip oil and mixtures thereof.

8. The use according to claim 7, **characterized in that** the oily vehicle comprises a mixture of avocado oil and rose hip oil.

9. The use according to claim 8, **characterized in that** the weight ratio of avocado oil/rose hip oil in the oily composition varies from 4:1 to 1:1.

10. The use according to any of the preceding claims, **characterized in that** the content of lipophilic *Hemerocallis* extract varies from 5 to 20 ppm.

11. The use according to any of the preceding claims, **characterized in that** the cosmetic composition further contains one or more secondary active ingredients selected from among an anti-ageing agent, acetyl-tetrapeptide-5, a plankton extract, maca extract, poppy petal extract, combination of extracts of Anchusa, poppy and Passiflora, an extract of *pancratium maritimum,* extract of *crocus discolor,* extract of mimosa seeds, extract of marigold petals, cocoa cells, vitamin C, vitamin E and hexylresorcinol.

12. The use according to any of the preceding claims, **characterized in that** the cosmetic composition is in the form of a gel, emulsion, biphasic oil-in-water or water-in-oil emulsion, body oil, mask, ointment, unguent, lotion, concentrated solution, nanocapsules or liposomes.

13. The use according to any of the preceding claims, **characterized in that** the cosmetic composition further comprises agents protecting against ultraviolet radiation or pigments forming an anti-ultraviolet screen.

14. Non-therapeutic cosmetic method to improve skin radiance and/or uniformize the complexion, consisting of applying to the skin regions concerned at least one topical cosmetic composition containing 0.01 to 2 % by weight of oily composition containing a lipophilic extract of *Hemerocallis fulva* and an oily vehicle comprising at least one vegetable oil.

15. Non-therapeutic use of a cosmetic composition for topical application comprising 0.01 to 2 % by weight of an oily composition containing a lipophilic extract of *Hemerocallis fulva* and an oily vehicle comprising at least one vegetable oil, to improve skin radiance and/or to uniformize the complexion.
